# EUROPEAN PATENT SPECIFICATION

(11) **EP 1 666 924 B1**
(45) Date of publication and mention of the grant of the patent: **10.12.2008**
(21) Application number: 06005509.2
(22) Date of filing: 23.08.2000
(51) Int. Cl.: A61L 27/34, G02B 1/04

(54) **Covalently-bound hydrophilic coating compositions for implants**
Kovalent gebundene, hydrophile Beschichtungszusammensetzungen für chirurgische Implantate
Compositions de revêtement hydrophiles à liaison covalente pour implants chirurgicaux

(30) Priority: 02.09.1999 US 152507 P
(43) Date of publication of application: 07.06.2006
(62) Divisional of application: 00961359.7
(73) Proprietor: Alcon, Inc., 6331 Hünenberg (CH)
(72) Inventor: Leboeuf, Albert R., Burleson, TX 76028 (US)
(74) Representative: Keller, Günter

(56) References cited:
- WO-A-95/11279
- WO-A-96/40303

## Description

### FIELD OF THE INVENTION

This invention relates to coatings for ophthalmic implants. In particular, the present invention relates to hydrophilic, covalently cross-linked copolymers that are covalently bound to the surface of ophthalmic implants.

### BACKGROUND OF THE INVENTION

Both rigid and foldable implantable ophthalmic lens materials are known. The most common rigid material used in ophthalmic implants is polymethyl methacrylate ("PMMA"). Foldable intraocular lens ("IOL") materials can generally be divided into three categories: silicone materials, hydrogel materials, and non-hydrogel ("hydrophobic") acrylic materials. See, for example, Foldable Intraocular Lenses, Ed. Martin et al., Slack Incorporated, Thorofare, New Jersey (1993). For purposes of the present application, hydrophobic acrylic materials are acrylic materials that absorb less than approximately 5% water at room temperature.

WO 96/40303 discloses soft, high refractive index ophthalmic lens materials. These materials consist of a copolymer comprising an aryl acrylic hydrophobic monomer and a hydrophilic monomer.

Silicone and non-hydrogel acrylic materials used in ophthalmic implants can potentially damage endothelial cells and perhaps other cells or tissues as well during or after the implant's insertion in the eye. These materials are generally hydrophobic and/or tacky and can pull cells off of eye tissues that contact the implant. Particularly in the case of phakic IOL's implanted between the capsular bag and the iris, there is significant potential for physical contact between the implant and surrounding cells or tissue even after the implant reaches its target location.

### SUMMARY OF THE INVENTION

The present invention relates to hydrophilic coating compositions for ophthalmic implants comprising silicone, hydrophobic acrylic or hydrogel materials. More specifically, the present invention relates to a copolymeric coating material for an implant where the copolymeric coating material comprises 2-phenylethyl (meth)acrylate and a hydrophilic monomer selected from the group consisting of hydroxyalkyl (meth)acrylates; polyethylene glycol (200 - 500) mono(meth)acrylates; and polyethylene glycol (200 - 500) monomethylether mono(meth)acrylates. The coating material is capable of absorbing from 40 to 70% water.

The present invention also relates to methods for applying a coating copolymer comprising 2-phenylethyl (meth)acrylate and a hydrophilic monomer as specified above to an implant's surface. In one embodiment, the method comprises dissolving the copolymer containing a latent cross-linking agent in a solvent to form a coating solution, contacting the coating solution with the implant's surface, and activating the latent cross-linking agent in the coating copolymer. In another embodiment, the method comprises dissolving the copolymer in a solvent to form a coating solution, adding a cross-linking agent to the coating solution, contacting the coating solution with the implant's surface, and heating the coated implant to generate cross-linking.

### DETAILED DESCRIPTION OF THE INVENTION

Unless indicated otherwise, all amounts are expressed as weight %.

The coating material of the present invention is a copolymer comprising 2-phenylethyl (meth)acrylate and a hydrophilic monomer selected from the group consisting of hydroxyalkyl (meth)acrylates; polyethylene glycol (200 - 500) mono(meth)acrylates; and polyethylene glycol (200 - 500) monomethylether mono(meth)acrylates. Preferred hydrophilic monomers are hydroxyalkyl (meth)acrylates. Most preferred are 2-hydroxyethyl methacrylate; 1,3-dihydroxypropyl methacrylate; 2,3-dihydroxypropyl methacrylate; mixtures of 1,3- and 2,3-dihydroxypropyl methacrylate ("GMMA"); monomethoxy glyceryl methacrylate; and mixtures thereof. The most preferred 2-phenylethyl (meth)acrylate is 2-phenylethyl methacrylate ("2-PEMA").

The coating material is capable of absorbing from about 40 to about 70% water, preferably from about 42 to about 55% water. The proportion of the copolymer's monomers will depend on the desired water content, with individual concentrations generally ranging from about 25 to about 60% for 2-phenylethyl (meth)acrylate and about 40 to about 75% for the hydrophilic monomer. In the most preferred embodiment, the desired water content is about 42 to about 55% and the coating material is comprises from 25 to 40% of 2-PEMA and from 25 to 35% of GMMA.

In one embodiment, in addition to the ingredients described above, the coating material also comprises a latent cross-linking agent, such as a blocked isocyanate. Suitable blocked isocyanate compounds include imidazole blocked isocyanatoethyl methacrylate. In this embodiment, the latent cross-linking agent is copolymerized with the other ingredients of the coating copolymer. In an alternative embodiment, the cross-linking agent is not added until the point where the coating copolymer is dissolved to form a coating solution. Examples of cross-linking agents that are suitable for use in this alternative embodiment include di-imidazole blocked 1,12-isocyanatododecane and peroxides, such as benzoyl peroxide and 2,4-dichlorobenzoyl peroxide.

The amount of the cross-linking agent contained in the coating compositions of the present invention will depend upon, among other factors, the chosen cross-linking agent and the degree of cross-linking desired. In general, the amount of the cross-linking agent necessary to cross-link the coating composition and secure it to the implant's surface will be about 0.5 - 3% for blocked isocyanates and about 3 - 6% for peroxides.

The copolymeric coating material is prepared by combining the chosen 2-phenylethyl (meth)acrylate, hydrophilic monomer and a polymerization initiator (optionally with a latent cross-linking agent) to form a coating composition and then curing the coating composition. Any type of polymerization initiator may be' used, including thermal initiators and photoinitiators, provided that the initiator can be activated without activating the latent cross-linking agent if present. Preferred initiators are UV- and blue-light activated initiators. The most preferred initiator is the benzoylphosphine oxide initiator, 2,4,6-trimethyl-benzoyldiphenylophosphine oxide ("TPO"), which is activated by blue-light. The amount of the polymerization initiator in the coating compositions of the present invention will depend upon the curing conditions. In general, however, the amount will be about 3 % (w/w) or less, preferably about 2 % (w/w) or less, and most preferably about 1 % (w/w).

In order to prevent premature cross-linking, the coating compositions of the present invention do not contain significant amounts of monomers having more than one unsaturated bond. Such ingredients include the common cross-linking monomers ethyleneglycol dimethacrylate; diethylene glycol dimethacrylate; ethyleneglycol diacrylate; allyl methacrylates; allyl acrylates; 1,3-propanediol dimethacrylate; 1,6-hexanediol dimethacrylate; 1,4-butanediol dimethacrylate; polyethyleneoxide diacrylates; and the like.

In addition to the 2-phenylethyl (meth)acrylate, hydrophilic monomer, any latent cross-linking agent, and polymerization initiator, the coating compositions optionally include one or more ingredients selected from the group consisting of UV absorbers that are copolymerizable with the 2-phenylethyl (meth)acrylate and hydrophilic monomer; blue-light blocking colorants that are copolymerizable with the 2-phenylethyl (meth)acrylate and hydrophilic monomer; reactive plasticizers to minimize haze or crazing; and chain transfer agents to retard cross-linking within the coating copolymer.

Ultraviolet absorbing chromophores can be any compound which absorbs light having a wavelength shorter than about 400 nm, but does not absorb any substantial amount of visible light. Suitable copolymerizable ultraviolet absorbing compounds are the substituted 2-hydroxybenzophenones disclosed in U.S. Patent No. 4,304,895 and the 2-hydroxy-5-acryloxyphenyl-2H-benzotriazoles disclosed in U.S. Patent No. 4,528,311. The most preferred ultraviolet absorbing compound is 2-(3'-methallyl-2'-hydroxy-5'-methyl phenyl) benzotriazole. Suitable polymerizable blue-light blocking chromophores include those disclosed in U.S. Patent No. 5,470,932. If a blue-light activated polymerization initiator is chosen and a blue-light blocking colorant is added, the polymerization initiator identity or concentration may have to be adjusted to minimize any interference.

Suitable reactive plasticizers or softening agents include polyethylene glycol (200 - 2000) mono(meth)acrylates and polyethylene glycol (200 - 2000) monomethylether mono(meth)acrylates. Methacrylates are preferred, with PEG(400)monomethylether monomethacrylate most preferred. If needed or desired, the amount of the reactive plasticizer will range from about 5 to about 25%. Depending on the implant's function and the thickness of the coating, some degree of haze or crazing may be tolerated such that a reactive plasticizer may not be required.

The chain transfer agent, if present, is typically added in an amount ranging from 0.01 to 0.4%. Many chain transfer agents are known in the art. Examples of suitable chain transfer agents include 1-dodecanethiol and 2-mercaptoethanol.

After the coating copolymer is polymerized, a coating solution is prepared by dissolving the coating copolymer in a solvent or mixture of solvents, such as a 50:50 (parts by weight) mixture of ethanol and 2-pentanone. The solvent or mixture of solvents is preferably chosen to give a clear, homogenous coating solution where the chosen solvent or solvent mixture does not evaporate so quickly that it leaves a hazy coating. If no latent cross-linking agent was included in the coating copolymer, a cross-linking agent, such as di-imidazole blocked 1,12-isocyanatododecane or a peroxide, is added to the coating solution.

The concentration of the coating copolymer in the coating solution will depend on the desired coating thickness. Other factors that will influence the thickness of the coating include the viscosity of the coating solution, the temperature of the coating solution and the implant, and the evaporation rate of the chosen solvent(s). In general, the coatings of the present invention will be no more than 1 µm thick, and preferably will be about 0.5 µm thick. A minimum coating thickness of about 0.01 µm is likely necessary to allow the coating to survive any manipulation of the implant (such as the folding of an IOL) and any abrasion caused during implantation or extended residence at the target site in a patient. A concentration of coating copolymer of about 7 - 9 % in the coating solution will typically produce a coating about 0.5 µm thick in a dip-coating process.

The coating solution is applied to the implant by conventional techniques, such as spin- or dip-coating processes. Dip-coating is preferred. The implant is preferably dipped quickly so as to minimize any swelling of the implant caused by the solvent in the coating solution.

After the coating is applied to the implant, the coating is dried. A two-stage drying process is preferred. First, the coated implant is allowed to dry in air until most or all of the solvent has evaporated (generally ≤ 15 minutes). Second, the coated implant is baked at elevated temperature, about 80 - 110 °C, to eliminate as much of the remaining solvent as possible and to activate the cross-linking agent. A preferred drying process involves room temperature air drying for 15 minutes, followed by baking at 100 °C for about 2 hours for blocked isocyanate cross-linking agents and 110 °C for about 3 - 5 hours for peroxide cross-linking agents.

Once the coating is secured to the implant's surface by covalent bonds formed by activating the cross-linking agent, the coating cannot be removed by solvents or solvent mixtures, including the same solvent used as the base in the preparation of the coating solution. After the coating is covalently-bound to the implant's surface, any impurities or unbound ingredients in the coating (or implant) may be removed by extraction in a suitable solvent, such as acetone in the case where the implant is an ACRYSOF® IOL. It is important that the swell response of the coating and the substrate to the extraction solvent not be too different in order to prevent damage to one or both during the extraction process. To prevent crazing or cracking of the coating, the swell of the coating should be greater than or equal to that of the substrate.

Before the coated implant is manipulated, the coating is preferably hydrated for several seconds to minimize crazing or other damage to the coating.

The implants suitable for coating with the hydrophilic coatings of the present invention are preferably made of hydrophobic acrylic materials, but could also be constructed of silicone, silicone-acrylic copolymers or hydrogels. Preferred hydrophobic acrylic materials are those described in U.S. Patent Nos. 5,290,892 and 5,693,095, the entire contents of which are hereby incorporated by reference. In the case where the implant is an IOL, the coatings of the present invention may be used in conjunction with substrate materials intended for use as a "hard" IOL (that is inserted in an unfolded state) or a "foldable" or "soft" IOL (that is inserted in a folded or compressed state). For example, the IOL material to be coated could be those IOL materials disclosed in U.S. Patent Nos. 5,693,095 or 5,331,073. The coating may be applied to the entire IOL or to only a portion of the IOL. As used herein, "implants" includes contact lenses.

In order to prepare the implant material to be coated so that it is capable of receiving the coating, it may be necessary or desirable to expose the surface to be coated to a reactive plasma gas prior to applying the coating composition of the present invention. Suitable reactive plasma gases include oxidizing gases, such as oxygen gas. A suitable plasma chamber is the P²CIM B-Series plasma chamber made by Advanced Plasma Systems, Inc. Using such a chamber, suitable plasma parameters include: power = 400 W, plasma gas = oxygen; pressure of the plasma gas = 30,0 Pa (225 mTorr); exposure time = 4 - 6 minutes.

The following examples are intended to be illustrative but not limiting.

### Examples 1 - 2 (latent cross-linking agent):

The formulations shown in Table 1 below were prepared and cured in polypropylene slab molds (10 mm x 20 mm x 0.9 mm). The formulations were cured by exposure to blue light for one hour using a Kulzer Palatray CU blue light unit (12 - 14 mW/cm²).

### Coating Solution Preparation

The coating copolymer of Example 1 was dissolved in a 21:3:2 (pbw) 2-pentanone:ethanol:dichloromethane solvent to give an 8.4% solution. The coating copolymer of Example 2 was dissolved in 2-pentanone to give an 8.0% solution. The resulting solutions were filtered through a Gelman glass fiber Acrodisc (1 µm) to give particulate-free coating solutions.

### Coating Application

A copolymer comprising 65% 2-phenylethyl acrylate; 30% 2-phenylethyl methacrylate; 1.8% o-methyl Tinuvin P; and 3.2% 1,4-butanediol diacrylate was prepared using 1.8% Perkadox-16 as a thermal initiator. This copolymer was cured in the slab molds described above, extracted in acetone for approximately 2 hours, dried in air at room temperature for about 1 hour, and then dried in an oven at 100 °C for about 1 hour. This material in the form of the defined slabs served as the implant/substrate material for all Examples ("the implant slabs").
The implant slabs were dipped in the coating solutions. Caution is taken to minimize the immersion time of the samples in the coating solution as the solvent will swell the sample. The coated implant was allowed to dry in air at room temperature for 15 minutes, followed by baking at 100 °C for 2 hours to activate the latent cross-linking agent and secure the coating to the implant's surface.

**TABLE 1 (all amounts in parts by weight)**

| INGREDIENT | 1 | 2 |
|---|---|---|
| 2-PEMA | 38.93 | 28.31 |
| 2-HEMA | --- | 34.66 |
| GMMA | 29.25 | --- |
| PEG (400) Monomethylether Monomethacrylate | 29.45 | 34.93 |
| Imidazole Blocked Isocyanatoethyl Methacrylate | 1.05 | 1.0 |
| 1-Dodecanethiol | 0.41 | 0.30 |
| Lucirin TPO | 0.89 | 0.78 |
| t-Butylperoctoate | | |
| | | |
| % water (slab) | 52.5 | 44.6 |
| Refractive Index (hydrated) | 1.425 | 1.429 |

### Example 3 (cross-linking agent added when coating solution is formed):

Coated implant slabs are prepared according to the procedure described above for Examples 1 - 2, except that the coating copolymer contains the ingredients shown in Table 2 below. The coating copolymer is cured using the Kulzer Palatray CU unit for one hour. A coating solution is formed by dissolving the coating copolymer in 2-pentanone to form an 8.0% solution. The implant slabs are dip coated and allowed to dry in air at room temperature for 15 minutes, followed by baking at 100 °C for 2 hours to de-block the isocyanate cross-linking agent and secure the coating to the implant's surface.

**TABLE 2 (all amounts in parts by weight)**

| INGREDIENT | 3 |
|---|---|
| 2-PEMA | 28.31 |
| 2-HEMA | 34.66 |
| GMMA | --- |
| PEG (400) Monomethylether Monomethacrylate | 34.93 |
| di-Imidazole Blocked 1,12-diisocyanatododecane | 1.0 |
| 1-Dodecanethiol | 0.30 |
| Lucirin TPO | 0.78 |
| t-Butylperoctoate | |
| | |
| % water (slab) | 44.6 |
| Refractive Index (hydrated) | 1.429 |

The embodiments described above are therefore considered to be illustrative in all respects and not restrictive, the scope of the invention being indicated by the appended claims rather than by the foregoing description.

## Claims

1. A coated ophthalmic implant comprising a coating and a substrate wherein the coating is attached to the substrate by covalent bonds, the coating is from 0.01 to 1 µm thick, and the coating comprises a covalently cross-linked copolymer comprising 2-phenylethyl (meth)acrylate, a hydrophilic monomer selected from the group consisting of hydroxyalkyl (meth)acrylates; polyethylene glycol (200 - 500) mono(meth)acrylates; and polyethylene glycol (200 - 500)monomethylether mono(meth)acrylates; and a cross-linking agent, and wherein the coating composition is capable of absorbing from 40 to 70% water.

2. The coated ophthalmic implant of Claim 1 wherein the substrate comprises a hydrophobic acrylic material.

3. A method of applying a coating to an ophthalmic implant comprising the steps of:
a) preparing an uncross-linked copolymer comprising 2-phenylethyl (meth)acrylate, a hydrophilic monomer selected from the group consisting of hydroxyalkyl (meth)acrylates; polyethylene glycol (200 - 500) mono(meth)acrylates; and polyethylene glycol (200 - 500) monomethylether mono(meth)acrylates; and optionally a latent cross-linking agent, such that the copolymer is capable of absorbing from 40 to 70% water;
b) forming a coating solution by dissolving the copolymer in an organic solvent and if the copolymer does not contain a latent cross-linking agent then adding a cross-linking agent to the solution;
c) applying the coating solution to the implant; and
d) drying the coating solution on the implant, such that the latent cross-linking agent or cross-linking agent is activated and the copolymer is covalently bound to the implant.

## Patentansprüche

1. Beschichtetes ophthalmisches Implantat, umfassend eine Beschichtung und ein Substrat, wobei die Beschichtung durch kovalente Bindungen an das Substrat gebunden ist, die Beschichtung 0,01 bis 1 µm dick ist und die Beschichtung ein kovalent vernetztes Copolymer, umfassend 2-Phenylethyl(meth)acrylat, ein hydrophiles Monomer, ausgewählt aus der Gruppe, bestehend aus Hydroxyalkyl(meth)acrylaten; Polyethylenglycol(200-500)mono(meth)-acrylaten und Polyethylenglycol(200-500)monomethylethermono(meth)acrylaten; und ein Vernetzungsmittel, umfaßt, und wobei die Beschichtungszusammensetzung 40 bis 70 % Wasser absorbieren kann.

2. Beschichtetes ophthalmisches Implantat nach Anspruch 1, wobei das Substrat ein hydrophobes Acrylmaterial umfaßt.

3. Verfahren zum Aufbringen einer Beschichtung auf ein ophthalmisches Implantat, umfassend die Schritte:
a) Herstellen eines unvernetzten Copolymers, umfassend 2-Phenylethyl(meth)acrylat, ein hydrophiles Monomer, ausgewählt aus der Gruppe, bestehend aus Hydroxyalkyl(meth)-acrylaten; Polyethylenglycol(200-500)mono(meth)acrylaten und Polyethylenglycol(200-500)monomethylethermono(meth)acrylaten; und gegebenenfalls ein latentes Vernetzungsmittel, so daß das Copolymer 40 bis 70 % Wasser absorbieren kann;
b) Bilden einer Beschichtungslösung durch Lösen des Copolymers in einem organischen Lösungsmittel, und wenn das Copolymer kein latentes Vernetzungsmittel enthält, dann Zugeben eines Vernetzungsmittels zu der Lösung;
c) Aufbringen der Beschichtungslösung auf das Implantat und
d) Trocknen der Beschichtungslösung auf dem Implantat, so daß das latente Vernetzungsmittel oder das Vernetzungsmittel aktiviert wird und das Copolymer kovalent an das Implantat gebunden wird.

## Revendications

1. Implant ophtalmique revêtu comprenant un revêtement et un substrat, dans lequel le revêtement est fixé au substrat par des liaisons covalentes, le revêtement a une épaisseur de 0,01 à 1 µm et le revêtement comprend un copolymère réticulé par covalence comprenant du (méth)acrylate de 2-phényléthyle, un monomère hydrophile choisi dans le groupe constitué de (méth)acrylates d'hydroxyalkyle ; de mono(méth)acrylates de polyéthylèneglycol (200-500) ; et de monométhyléther mono(méth)acrylates de polyéthylèneglycol (200-500) ; et un agent de réticulation, et dans lequel la composition de revêtement est capable d'absorber 40 à 70 % d'eau.

2. Implant ophtalmique revêtu selon la revendication 1, dans lequel le substrat comprend un matériau acrylique hydrophobe.

3. Procédé d'application d'un revêtement à un implant ophtalmique, comprenant les étapes suivantes :
a) on prépare un copolymère non réticulé comprenant du (méth)acrylate de 2-phényléthyle, un monomère hydrophile choisi dans le groupe constitué de (méth)acrylates d'hydroxyalkyle ; de mono(méth)acrylates de polyéthylèneglycol (200-500) ; et de monométhyléther-mono(méth)acrylates de polyéthylèneglycol (200-500) ; et éventuellement un agent de réticulation latent, de sorte que le copolymère soit capable d'absorber 40 à 70 % d'eau ;
b) on forme une solution de revêtement en dissolvant le copolymère dans un solvant organique et, si le copolymère ne contient par d'agent de réticulation latent, on ajoute ensuite un agent de réticulation à la solution ;
c) on applique la solution de revêtement à l'implant ; et
d) on sèche la solution de revêtement sur l'implant, de sorte que l'agent de réticulation latent ou l'agent de réticulation soit activé et que le copolymère soit lié par covalence à l'implant.
